Europäisches Patentamt

European Patent Office

Office européen des brevets

(11) Publication number: **0 242 784**
A1

# EUROPEAN PATENT APPLICATION

(21) Application number: 87105571.1

(22) Date of filing: 15.04.87

(51) Int. Cl.³: **B 01 D 13/02**
**C 07 C 31/18**

(30) Priority: 18.04.86 SE 8601772

(43) Date of publication of application:
28.10.87 Bulletin 87/44

(84) Designated Contracting States:
AT BE CH DE ES FR GB IT LI NL

(71) Applicant: Perstorp AB
Box 5000
S-284 00 Perstorp(SE)

(72) Inventor: Holmberg, Hans V.
Tullgatan 8
S-223 54 Lund(SE)

(72) Inventor: Larsson, Hans-Erik
Magnarp 666
S-262 00 Ängelholm(SE)

(74) Representative: Hansen, Bernd, Dr.rer.nat. et al,
Hoffmann, Eitle & Partner Patentanwälte Arabellastrasse 4
D-8000 München 81(DE)

(54) Method in the production of a polyalcohol.

(57) Method in the production of a polyalcohol, such as pentaerythritol, trimethylolpropane, neopentylglycol and the like in the presence of sodium hydroxide or calcium hydroxide, wherein the sodium formiate respectively calcium formiate solution formed at the production of the polyalcohol is allowed to pass a device comprising bipolar membranes in combination with conventional ion exchanging membranes arranged side by side in a suitable order to a so-called electrodialysis stack with different kinds of compartments and with an anode at one side and a cathode at the other side of the stack. The sodium formiate respectively calcium formiate in the solution is split by means of the membranes when a current is passing between the anode and the cathode and separated in different solutions in the compartments.

EP 0 242 784 A1

## Method in the production of a polyalcohol

The present invention relates to a method in the production of a polyalcohol, such as pentaerythritol, trimethylolpropane, neopentylglycol and the like.

In the conventional production of this kind of polyalcohols sodium hydroxide or calcium hydroxide is added. Said hydroxide is then transformed in the production process to sodium formiate respectively calcium formiate.

In order to get a good production economy the formiate has so far been recovered in different ways. However, this is complicated and considerably increases investment and operation costs.

Therefore, efforts have been made for a long time to find a method in the production of polyalcohols, where the expensive handling of formiate can be avoided.

Quite unexpectedly the solution of the problem mentioned has been found according to the present invention and method in the production of a polyalcohol, such as pentaerythritol, trimethylolpropane, neopentylglykol or the like in the presence of sodium formiate respectively calcium formiate has been brought about.

The sodium formiate respectively calcium formiate solution formed in the production of the polyalcohol is allowed to pass a device comprising bipolar membranes in combination with conventional ion exchanging membranes arranged side by side in a suitable order to a so-called electrodialysis stack with different kinds of compartments and with an anode at one side and a cathode at the other side of the stack. The sodium formiate respectively calcium formiate in the solution is split by means of the membranes when a current is passing between the anode and the cathode, and separated in different solutions in the compartments.

In addition to the bipolar membranes, preferably anion as well as cation exchanging membranes are included in the electrodialysis stack.

Then three kinds of compartments are formed and a separation in three different solutions is obtained in the compartments; a first solution containing sodium hydroxide respectively calcium hydroxide; a second solution containing formic acid and a third solution containing sodium formiate respectively calcium formiate.

According to another embodiment of the invention the electrodialysis stack contains only bipolar membranes and cation exchanging membranes. Then two kinds of compartments are formed and a separation in two different solutions is obtained in the compartments; a first solution containing sodium hydroxide respectively calcium hydroxide and a second solution containing formic acid and sodium formiate respectively calcium formiate.

Advantageously, the sodium hydroxide respectively calcium hydroxide obtained can be recirculated to the reactor for production of polyalcohol. In this way, the need for new hydroxide in the process is decreased, which of course reduces the costs for the process.

The formic acid obtained according to the first embodiment above is removed from the process and can be sold for different purposes.

In addition to the above mentioned polyalcohols the invention can also be used in the production of trimethyloletan, dimethylol-propionic acid, trimethylolacetic acid, dimethylolbutanol, tetramethylolcyclohexanol and anhydroenneaheptitol for instance.

Suitably, the electrodialysis treatment is carried out on the so-called mother liquor when the polyalcohol has been separated from it. However, it is possible to arrange the electrodialysis at another step of the production. One suitable place is directly after the reactor. Then polyalcohol as well as by-products are present in the reaction mixture.

The invention will be explained further in connection with the embodiment examples below and the figures enclosed.

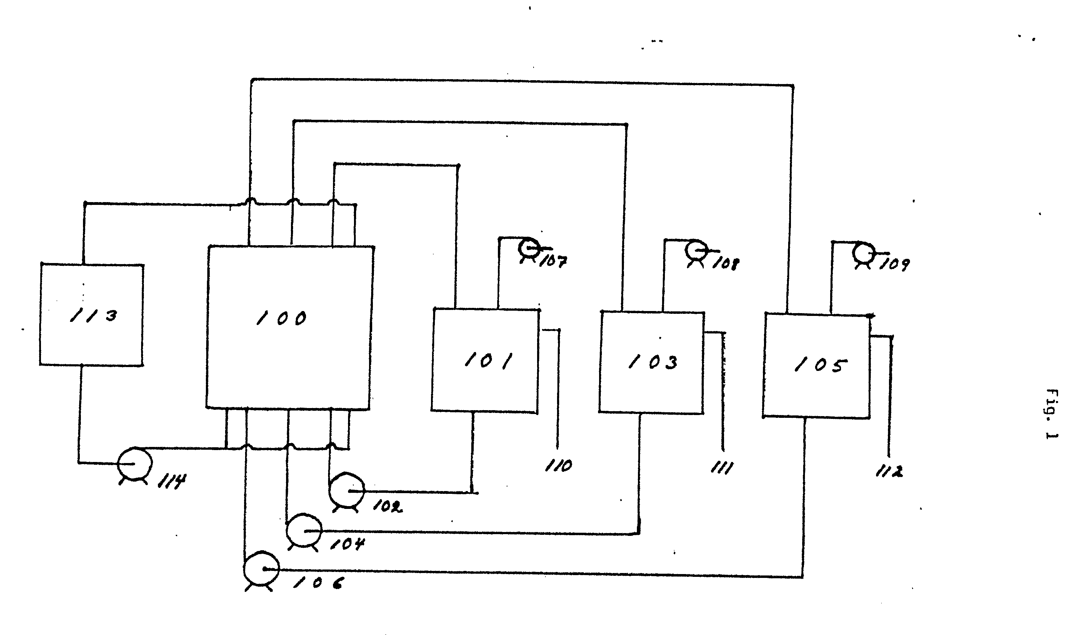

The embodiment examples show a treatment of a reaction mixture from the production of pentaerythritol by electrodialysis according to the invention, whereby formic acid and sodium hydroxide are obtained. Fig. 1 shows a schematic disposition of one embodiment of an electrodialysis construction according to the invention.

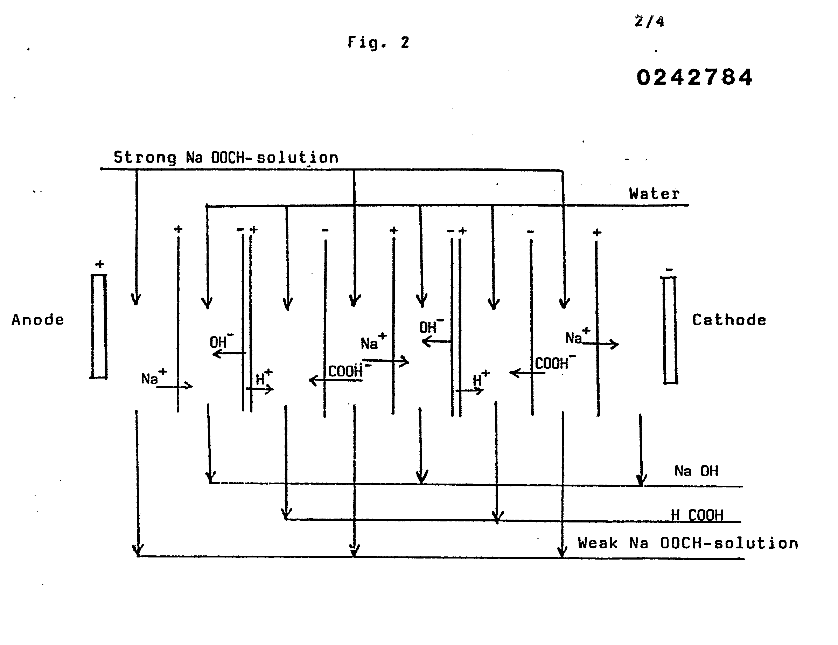

Fig. 2 illustrates a schematic construction of an electrodialysis device according to the invention with the different ions and currents drawn.

Fig. 3 shows a schematic construction of an electrodialysis device with a larger number of compartments than in the device according to Fig. 2.

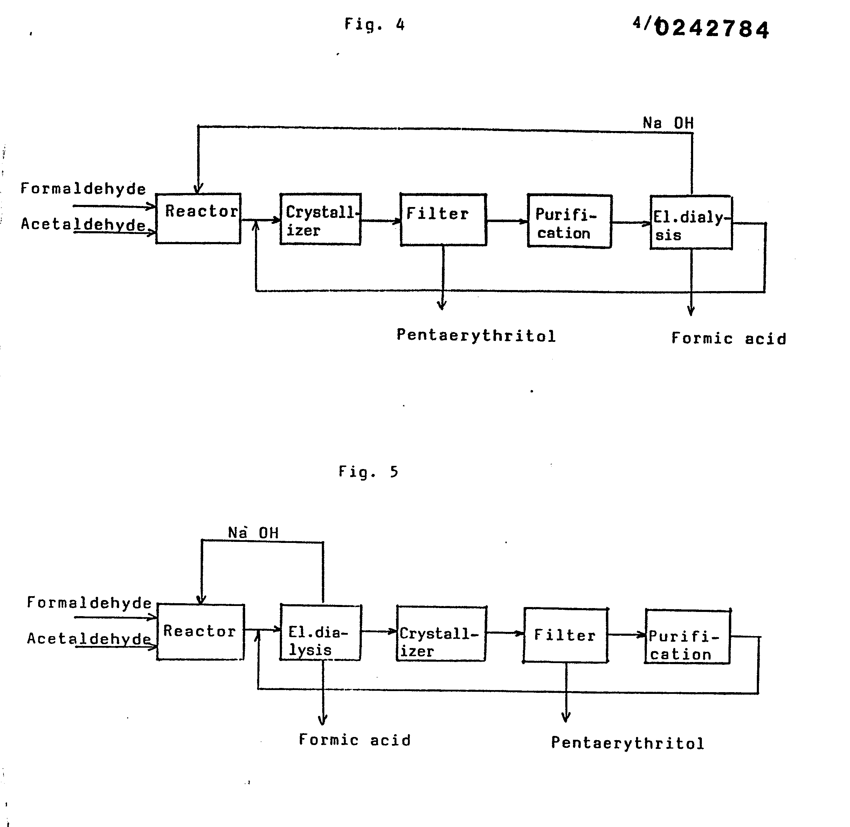

Figures 4 and 5 show examples of how the electrodialysis device can be placed in a pentaerythritol process.

Example

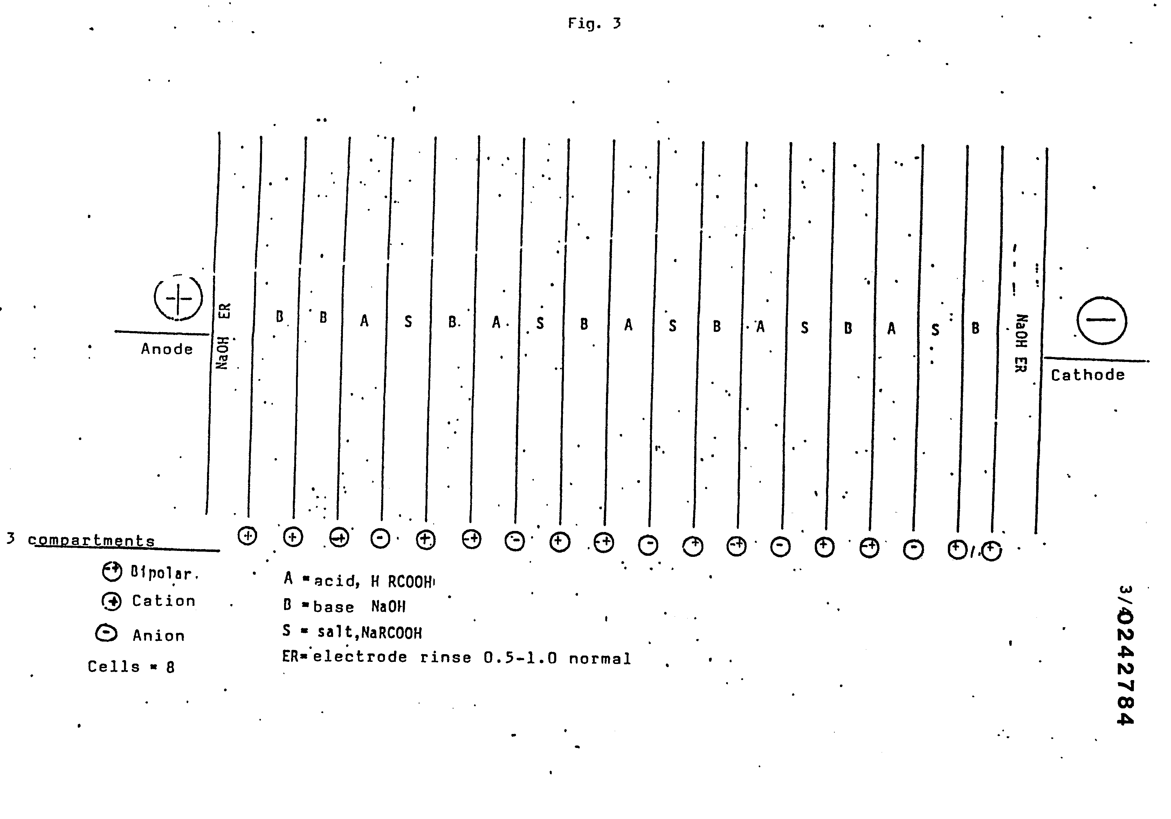

An electrodialysis cell 100 (Fig. 1) was constructed according to the enclosed Figures 1 and 3 and formed 19 compartments; 1 anolyte, 2 base compartments, 5 repeating units for acid, salt and base (15 compartments totally) and finally 1 catholyte. The compartments were separated by ion exchanging membranes in the following way: Cation exchanging membranes between anolyte and base, base and base, salt and base and between base and catholyte; anion exchanging membranes between acid and salt and bipolar membranes between base and acid. The bipolar membranes were placed with the cation exchanging side facing the acid compartments. The anode was made of nickel and the cathode of stainless steel.

Solution was circulated to the base compartments from a base reservoir 101 and back to the resevoir by means of a pump 102.

In a similar manner a separate electrolyte reservoir 113 and a pump 114 were used to circulte a solution to the anolyte and catholyte compartments. Solution was circulated to the acid compartments from a reservoir 103 by means of a pump 104. The concentration of acid and base was regulated by adding more solution or water to the acid and base reservoir by means of two metering pumps, 107 and 108. This addition caused an overflow from the acid and base reservoir through overflow tubes 110 and 111. A salt solution was circulated through the salt compartments from the salt reservoir 105 by means of a pump 106. A reaction mixture from pentaerythritol production containing 25 percent sodium formiate was continuously fed into the salt reservoir by a metering pump 109, which caused an overflow of salt solution through an overflow tube 112. This salt solution could be recycled to the process.

The electrolyte and base reservoirs were charged with sodium hydroxide and the acid reservoir with a solution of formic acid in water. The pumps connected to reservoirs were turned on and direct current of 13 A was allowed to pass.

When equilibrium was reached the overflowing solutions were analyzed. The recycled reaction mixture contained 12 % sodium formiate. At the same time the salt splitting process delivered a 10 % sodium hydroxide solution and a 25 % formic acid solution.

The invention is not limited to the embodiments shown, since these can be modified in different ways within the scope of the invention.

CLAIMS

1. Method in the production of a polyalcohol, such as pentaerythritol, trimethylolpropane, neopentylglycol and the like in the presence of sodium hydroxide or calcium hydroxide, wherein the sodium formiate respectively calcium formiate solution formed in the production of the polyalcohol is allowed to pass a device comprising bipolar membranes in combination with conventional ion exchanging membranes arranged side by side in a suitable order to a so-called electrodialysis stack with different kinds of compartments and with an anode at one side and a cathode at the other side of the stack, whereby the sodium formiate respectively calcium formiate in the solution is split by means of the membranes when a current is passing between the anode and the cathode and separated in different solutions in the compartments.

2. Method according to claim 1, wherein anion as well as cation exchanging membranes are used, whereby three kinds of compartments are formed and a separation in three different solutions is obtaied in the compartments; a first solution containing sodium hydroxide respectively calcium hydroxide, a second solution containing formic acid and a third solution containing sodium formiate respectively calcium formiate.

3. Method according to claim 1, wherein the electrodialysis stack contains only bipolar membranes and cation exchanging membranes, whereby two kinds of compartments are formed and a separation in two different solutions is obtained in the compartments; a first solution containing sodium hydroxide respectively calcium hydroxide and a second solution containing formic acid and sodium formiate respectively calcium formiate.

4. Method according to any one of claims 1-3, wherein the sodium hydroxide respectively calcium hydroxide obtained is recycled to the reactor for production of polyalcohol.

5. Method according to any one of claims 1-4, wherein the electrodialysis treatment is carried out on the so-called mother liquor when the polyalcohol has been separated from it.

6. Method according to any one of claims 1-4, wherein the electrodialysis treatment is caried out on the mother liquor before the polyol has been separated.

Fig. 1

0242784

Fig. 2

Fig. 3

Anode  ⊕

NaOH ER

B B A S B A S B A S B A S B A S B A S B A S B

NaOH ER

Cathode  ⊖

3 compartments

⊕ Bipolar
⊕ Cation
⊖ Anion

Cells = 8

A = acid, H RCOOH
B = base  NaOH
S = salt, NaRCOOH
ER = electrode rinse 0.5-1.0 normal

3/0242784

Fig. 4          4/0242784

Fig. 4

Fig. 5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | US-A-2 865 822 (C.R. MURPHY) * Column 1, lines 15-23; column 1, line 62 - column 3, line 28; figures 1,2 * | 1-4,6 | B 01 D 13/02 C 07 C 31/18 |
| Y | CHEMICAL ENGINEERING, vol. 93, no. 5, March 1986, pages 42-43, New York, US; N. BASTA: "Use electrodialytic membranes for waste recovery" * Whole document * | 1-4,6 | |
| A | US-A-2 829 095 (KENICHI ODA et al.) * Column 1, line 15 - column 2, line 2; figure * | 1,2 | |
| A | US-A-3 779 883 (A.H. HEIT) * Abstract; figure; column 2, line 66 - column 4, line 8 * | 1 | TECHNICAL FIELDS SEARCHED (Int Cl.4) B 01 D C 25 B C 07 C |

The present search report has been drawn up for all claims

| Place of search THE HAGUE | Date of completion of the search 15-07-1987 | Examiner VAN IDDEKINGE R.E. |
|---|---|---|